# FASCICULE DE BREVET EUROPEEN

(11) **EP 1 117 454 B1**
(45) Date de publication et mention de la délivrance du brevet: **17.11.2004**
(21) Numéro de dépôt: 99946266.6
(22) Date de dépôt: 30.09.1999
(51) Int. Cl.: A61M 5/32, A61M 5/28

(54) **DISPOSITIF D'INJECTION A USAGE UNIQUE DESTINE A ETRE PRE-REMPLI**
VORGEFÜLLTE INJEKTIONSVORRICHTUNG FÜR EINMALVERWENDUNG
DISPOSABLE INJECTION DEVICE DESIGNED TO BE PRE-FILLED

(30) Priorité: 01.10.1998 FR 9812308
(43) Date de publication de la demande: 25.07.2001
(73) Titulaire: Sanofi-Aventis, 75013 Paris (FR)
(72) Inventeur: Brunel, Marc, 31000 Toulouse (FR)
(74) Mandataire: Cabinet BARRE LAFORGUE & associés
(86) Numéro de dépôt international: PCT/FR1999/002325
(87) Numéro de publication internationale: WO 2000/020059

(56) Documents cités:
- WO-A-85/04590
- US-A- 3 916 893
- US-A- 5 667 494

## Description

L'invention concerne un dispositif d'injection du type à usage unique conçu pour être pré-rempli d'une dose de liquide, notamment médicamenteux, à injecter.

Les dispositifs d'injection à usage unique destinés à être pré-remplis comportent un corps de seringue, soit doté d'une embase dans laquelle est scellée une aiguille protégée par un capuchon de protection, soit comportant un raccord conique mâle à verrouillage permettant d'adapter sur ledit corps de seringue un raccord conique femelle à verrouillage portant une aiguille d'injection protégée par un capuchon de protection, lesdits raccords coniques définissant un assemblage communément connu sous l'appellation "assemblage conique LÜER".

Les dispositifs d'injection les plus classiques dits à "aiguille humide" sont du type doté d'un corps de seringue comportant une embase dans laquelle est scellée une aiguille d'injection obturée au moyen d'un capuchon de protection en élastomère doté d'un alésage borgne interne ménagé dans le fond dudit capuchon, à l'intérieur duquel vient se loger en force l'extrémité de ladite aiguille de façon à garantir l'étanchéité du dispositif d'injection avant injection.

Le premier inconvénient de tels dispositifs d'injection réside dans le fait qu'ils imposent, lors de la mise en place du capuchon de protection, de centrer parfaitement l'aiguille d'injection par rapport à l'alésage dudit capuchon. Or, dans la pratique, ce centrage s'avère parfois approximatif, de sorte que le montage du capuchon conduit fréquemment à détériorer ledit capuchon ou l'aiguille, avec pour conséquence des rebuts non négligeables de fabrication.

De plus, selon ce principe, la qualité de l'aiguille (affûtage, siliconage) se trouve systématiquement affectée du fait des frottements de la pointe de ladite aiguille contre la paroi interne de l'alésage borgne du capuchon lors du montage en force de cette dernière.

Enfin, le liquide renfermé dans ces dispositifs d'injection se trouve obligatoirement au contact des matériaux constituant l'aiguille d'injection et le capuchon de protection qui peuvent, pour certains types de liquide, affecter la conservation de ces derniers.

Pour pallier ces inconvénients, il a été conçu de multiples dispositifs d'injection dits "à aiguille sèche", pour lesquels l'aiguille d'injection se trouve isolée du liquide renfermé dans le corps de seringue jusqu'au moment de l'injection.

Un premier type de dispositif d'injection "à aiguille sèche" est celui utilisé couramment dans le domaine dentaire et comporte un flacon renfermant le liquide à injecter et obturé par une membrane, et une aiguille bi-pointe apte à être déplacée axialement par rapport audit flacon, de façon à venir percer la membrane au moment de l'injection. De tels dispositifs d'injection sont notamment décrits dans les brevets DE-847473, FR-2347055, US-4639250, EP-602883, DE-2008751, DE-1909794.

Les inconvénients de ce type de dispositif d'injection sont de deux ordres. En effet, et en premier lieu, le fait de nécessiter une aiguille bi-pointe conduit à un surcoût concernant le prix de revient de ces dispositifs d'injection, résultant d'une part du coût en lui-même de ladite aiguille, et d'autre part, de l'obligation d'effectuer deux opérations d'affûtage en lieu et place de la seule opération d'affûtage requise pour une aiguille classique. De plus, il arrive fréquemment, notamment pour des petits diamètres d'aiguille, que l'on se trouve confrontés à des problèmes de carottage conduisant à l'inclusion de particules de membrane à l'intérieur de la lumière de l'aiguille, qui soit obturent cette lumière, soit sont injectés avec le liquide.

Un deuxième type de dispositif d'injection à "aiguille sèche", comporte un corps de seringue logeant deux bouchons délimitant la chambre renfermant le liquide, et sur lequel est sertie une embase, ledit corps de seringue possédant, en outre, un compartiment doté d'un conduit de communication avec l'aiguille d'injection, ménagé de façon à être mis en relation avec la chambre uniquement après déplacement axial des pistons.

De tels dispositifs d'injection, notamment décrits dans les brevets FR-2412320, FR-2208684, EP-191508, EP-588148 et EP-720857, permettent de pallier les inconvénients des dispositifs d'injection ci-dessus décrits. Toutefois, ils présentent également eux-mêmes deux inconvénients. En effet, et en premier lieu, l'opération de sertissage de l'embase sur le corps de seringue s'avère délicate et exige une attention particulière en vue de garantir une parfaite étanchéité entre ladite embase et ledit corps de seringue. De plus et surtout, de tels dispositifs d'injection peuvent être sujets à des écoulements accidentels du liquide renfermé dans la chambre, résultant par exemple d'une dilatation du volume de gaz renfermé dans ladite chambre, ou d'une dépressurisation notamment lors d'un transport aérien, qui conduisent à provoquer le déplacement axial du bouchon d'accès au compartiment d'évacuation du liquide.

Un troisième type de dispositif d'injection à "aiguille sèche" décrit notamment dans les brevets US-A-3 916 893, EP-150681, EP-111796, FR-2330413 et WO-8404252, permet de pallier l'ensemble des inconvénients sus-évoqués. A cet effet, ces dispositifs d'injection comportent, d'une part, un corps de seringue doté d'une chambre obturée par un bouchon en caoutchouc percé d'un alésage longitudinal traversant, et d'autre part, une embase mobile axialement à l'intérieur de l'alésage dudit bouchon, dotée de conduits ménagés de façon à mettre en communication l'aiguille d'injection et la chambre lors d'un déplacement axial de ladite embase tendant à l'enfoncer dans le bouchon.

La présente invention vise un dispositif d'injection de conception similaire à celle des dispositifs d'injection du troisième type décrit ci-dessus, et a pour principal objectif de fournir un dispositif d'injection ergonome alliant les avantages de ces dispositifs d'injection (étanchéité, garantie contre les risques d'écoulement accidentel...) et dont l'activation en vue d'une injection s'opère de façon très simple par un geste très naturel.

A cet effet, l'invention vise un dispositif d'injection du type décrit dans les quatre brevets ci-dessus cités, et comprenant donc un corps de seringue délimitant une chambre destinée à être remplie d'un liquide, notamment médicamenteux, et un organe d'obturation de la chambre et de distribution du liquide, comportant un embout d'obturation de ladite chambre, d'une part prolongé d'une embase dans laquelle est scellée une aiguille coiffée par un capuchon de protection, et d'autre part, percé dans le prolongement de ladite aiguille d'un conduit de distribution du liquide, ledit organe d'obturation et de distribution étant mobile axialement entre une position, dite position d'obturation, où le conduit de distribution est isolé de la chambre du corps de seringue, et une position, dite position d'injection, où ledit conduit de distribution communique avec ladite chambre.

Selon l'invention, ce dispositif d'injection se caractérise en ce que :
- l'organe d'obturation et de distribution comprend un tronçon intermédiaire disposé entre l'embout d'obturation et l'embase,
- il comprend une bague dotée d'une paroi frontale percée d'un orifice axial pour le passage du capuchon de protection, ladite bague étant adaptée pour coiffer un des tronçons d'extrémité du corps de seringue et pour délimiter, dans le prolongement dudit tronçon d'extrémité, deux compartiments internes juxtaposés axialement, chacun de forme conjuguée du collet intermédiaire de l'organe d'obturation et de distribution, apte à loger ledit collet intermédiaire, lesdits compartiments étant séparés par des organes de butée axiale aptes à autoriser un déplacement axial du collet intermédiaire d'un compartiment vers l'autre compartiment,
- la bague et le collet intermédiaire comprennent des organes conjugués, respectivement interne et externe, de blocage relatif en rotation desdites bague et collet intermédiaire,
- le capuchon comprend un tronçon d'extrémité adapté pour s'étendre au travers de l'orifice axial de la paroi frontale de la bague, de façon à coiffer l'embase et à venir axialement en butée contre le collet intermédiaire de l'organe d'obturation et de distribution,
- le tronçon d'extrémité du capuchon et le collet intermédiaire de l'organe d'obturation et de distribution présentent frontalement, en regard, des profils conjugués en forme de came aptes à engendrer un déplacement axial de l'organe d'obturation et de distribution de sa position d'obturation vers sa position d'injection, lors d'un mouvement de rotation du capuchon de protection,
- et le capuchon comporte des organes externes agencés pour venir en butée axiale sur la paroi frontale de la bague, dans une position où ledit capuchon coiffe l'embase de l'organe d'obturation et de distribution.

L'activation d'un tel dispositif d'injection, en vue de l'injection, résulte donc d'une simple rotation du capuchon de protection provoquant un déplacement axial de l'organe d'obturation et de distribution conduisant à mettre en communication l'aiguille et la chambre du corps de seringue.

Cette activation nécessite donc simplement d'effectuer un geste naturel, réalisé de façon classique en vue du retrait d'un capuchon de protection, qui consiste à imprimer audit capuchon de protection un mouvement de rotation.

De plus, le déplacement axial de l'organe d'obturation et de distribution engendré par cette rotation du capuchon de protection est irréversible, du fait que tout déplacement ultérieur inverse s'avère par la suite interdit.

Selon un mode de réalisation avantageux de l'invention, chaque profil en forme de came présente deux pentes s'étendant symétriquement de part et d'autre d'un axe de symétrie longitudinal, de façon à autoriser une rotation du capuchon de protection dans l'un ou l'autre sens de rotation.

Ainsi, l'activation du dispositif d'injection peut être obtenue indifféremment en faisant tourner le capuchon de protection dans l'un ou l'autre sens de rotation.

Selon un autre mode de réalisation avantageux de l'invention, les profils en forme de came du collet intermédiaire de l'organe d'obturation et de distribution et du tronçon d'extrémité du capuchon de protection présentent respectivement une forme concave et une forme convexe conjuguées.

Selon un autre mode de réalisation avantageux de l'invention, le tronçon d'extrémité du capuchon de protection comporte un pion externe radial au niveau d'au moins un des profils en forme de came.

De plus, la bague comporte alors, pour chaque pion externe du capuchon de protection, une échancrure ménagée sur le pourtour de l'orifice axial de la paroi frontale, ladite échancrure étant ménagée de façon à être décalée angulairement d'un angle (α) par rapport au pion externe, dans la position d'obturation de l'organe de distribution et d'obturation, et pour se trouver à l'aplomb dudit pion externe dans la position d'injection dudit organe d'obturation et de distribution.

Selon cette disposition, le capuchon de protection ne peut être retiré sans avoir subi au préalable un mouvement de rotation permettant d'amener chaque pion externe à l'aplomb d'une échancrure ménagée dans la paroi frontale de la bague, mouvement de rotation au cours duquel, tel que précité, l'organe d'obturation et de distribution est déplacé axialement de façon automatique.

Le mouvement à imprimer au capuchon de protection en vue du retrait de celui-ci, correspond donc ainsi exactement au mouvement classique imprimé à tout capuchon de protection en vue de son retrait, à savoir un mouvement de rotation conjugué à un effort de traction exercé sur ledit capuchon de protection.

Selon un autre mode de réalisation avantageux de l'invention, le capuchon de protection comporte, pour chaque échancrure de la paroi frontale de la bague, une ailette longitudinale décalée angulairement d'un angle (α) par rapport à chaque pion externe dudit capuchon de protection, apte à pouvoir coulisser dans ladite échancrure lorsque ce capuchon de protection est présenté en regard de l'intérieur de la bague.

La présence de cette (ou de ces) ailette(s) permet d'assembler dans une phase préalable l'organe d'obturation et de distribution et le capuchon de protection, puis de monter cet ensemble en l'introduisant à l'intérieur de la bague, montage au cours duquel la position angulaire de chaque pion externe du capuchon est automatiquement indexée grâce à la disposition de la (ou des)dite(s) ailette(s).

En outre, et de façon préférentielle, chaque ailette longitudinale du capuchon de protection est interrompue à distance de l'extrémité du tronçon d'extrémité dudit capuchon de protection, et présente une épaisseur adaptée pour venir en butée axiale sur la paroi frontale de la bague.

De plus, selon un autre mode de réalisation avantageux de l'invention, chaque ailette du capuchon de protection comprend une face externe latérale inclinée longitudinalement de façon à former une rampe apte à permettre à ladite ailette de franchir l'échancrure de la paroi frontale de la bague en fin d'introduction dudit capuchon de protection à l'intérieur de ladite bague.

Cette forme des ailettes permet de monter l'ensemble organe d'obturation et de distribution/capuchon de protection à l'intérieur de la bague tel que précédemment décrit, tout en conférant auxdites ailettes la fonction de butée axiale dudit capuchon de protection sur la paroi frontale de la bague, une fois ce montage effectué.

Selon un autre mode de réalisation avantageux de l'invention :
- la bague délimite un logement interne de forme cylindrique et comporte au moins une rainure interne longitudinale par rapport au fond de laquelle fait saillie un cran ménagé en position intermédiaire le long de ladite rainure, ledit cran présentant longitudinalement une face externe en forme de dièdre apte à faire office d'organe de butée axiale séparant les deux compartiments internes de ladite bague,
- le collet intermédiaire de l'organe d'obturation et de distribution comprend, pour chaque rainure de la bague, un ergot latéral apte à venir se loger dans ladite rainure, et de forme conjuguée des compartiments délimités par le cran s'étendant dans cette dernière.

Par ailleurs, le dispositif d'injection selon l'invention comprend avantageusement un bouchon d'obturation de dimensions adaptées pour être inséré dans le corps de seringue, percé d'un alésage traversant débouchant dans ledit corps de seringue au niveau d'un lamage.

De plus, dans ce cas, l'organe d'obturation et de distribution présente des dimensions adaptées pour être inséré dans l'alésage du bouchon d'obturation, et comprend un conduit de distribution comportant une branche transversale ménagée de façon à être positionnée en retrait du lamage dudit bouchon d'obturation, dans la position d'obturation de l'organe d'obturation et de distribution, et pour s'étendre dans ledit lamage dans la position d'injection dudit organe.

D'autres caractéristiques, buts et avantages de l'invention ressortiront de la description détaillée qui suit en référence aux dessins annexés qui en représentent à titre d'exemple non limitatif un mode de réalisation préférentiel. Sur ces dessins :
- la figure 1 est une vue en perspective avec un arraché partiel d'un dispositif d'injection conforme à l'invention,

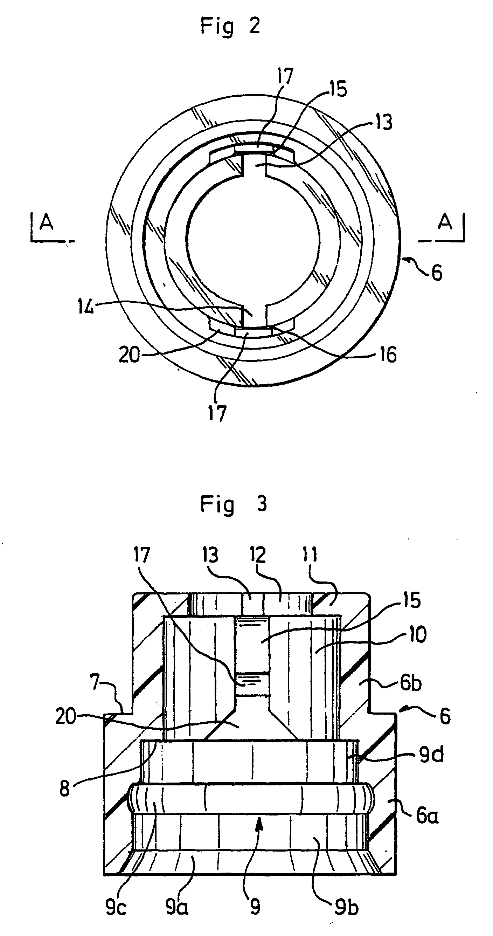

- la figure 2 est une vue frontale de dessous de la bague du corps de seringue de ce dispositif d'injection,
- la figure 3 est une coupe longitudinale par un plan axial A de cette bague,

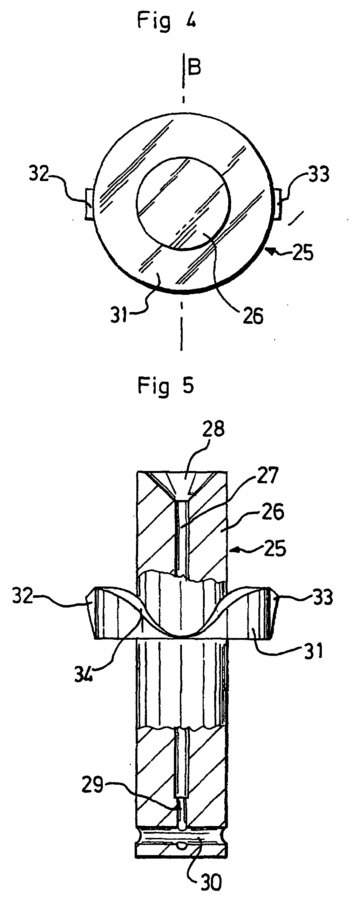

- la figure 4 est une vue frontale de dessous de l'organe d'obturation et de distribution de ce dispositif d'injection,
- la figure 5 est une vue longitudinale partiellement en coupe par un plan axial B de cet organe d'obturation et de distribution,

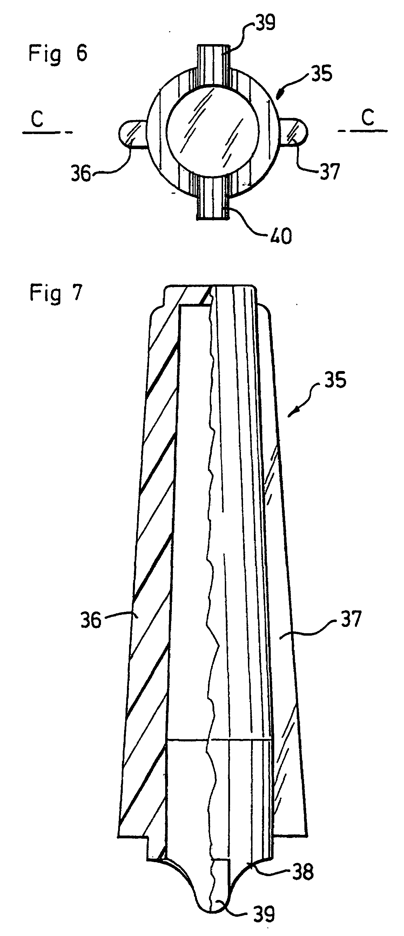

- la figure 6 est une vue frontale de dessous du capuchon de protection de ce dispositif d'injection,
- la figure 7 est une vue longitudinale partiellement en coupe par un plan axial C, de ce capuchon de protection,
- les figures 8 et 9 sont des vues longitudinales de l'intérieur de ce dispositif d'injection respectivement dans sa position d'obturation, et sa position d'injection avant retrait du capuchon de protection, sur lesquelles l'organe d'obturation et de distribution et ledit capuchon de protection sont représentés partiellement en coupe par un plan axial D,
- et la figure 10 est une coupe longitudinale par le plan axial D de ce dispositif d'injection dans sa position d'injection, après retrait du capuchon de protection.

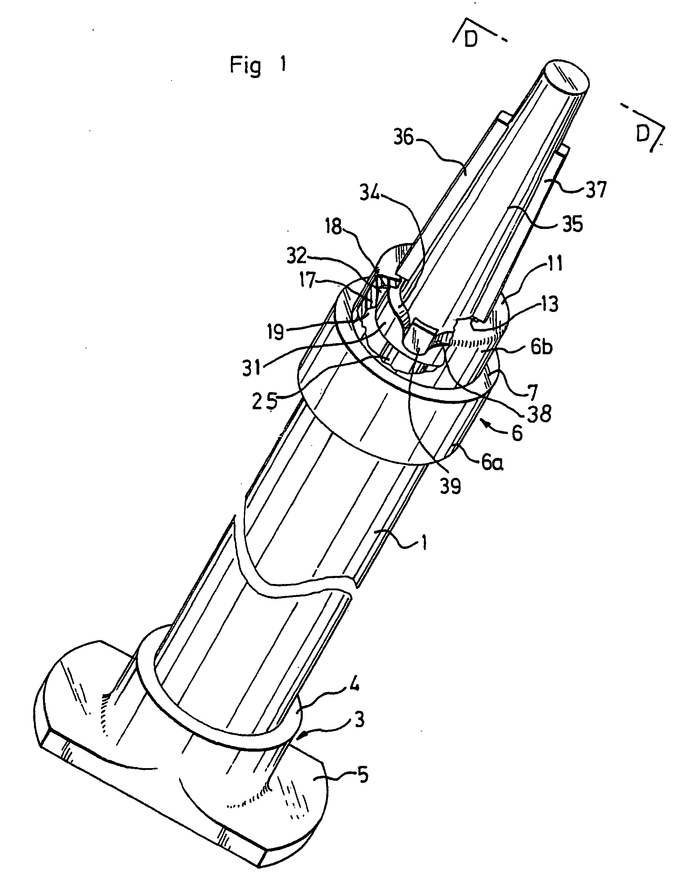

Le dispositif d'injection représenté à la figure 1 est du type à usage unique, c'est-à-dire destiné à être rempli d'un liquide à injecter. Il est à noter que bien que le dispositif d'injection tel que représenté ne possède pas d'étui protecteur destiné à permettre de protéger l'aiguille sans risque de piqûre, après injection, il peut être équipé, de façon connue en soi, de tout étui protecteur de type classique remplissant cette fonction.

Ce dispositif d'injection comprend, en premier lieu, un corps de seringue classique comportant un tube cylindrique 1 doté d'une collerette externe 2 au niveau d'une de ses extrémités.

Ce corps de seringue comprend, en outre, un embout 3 adapté pour venir s'adapter classiquement sur le tronçon d'extrémité du tube cylindrique 1 opposé à la collerette 2, et comportant de façon traditionnelle un manchon 4 adapté pour s'emmancher sur ladite extrémité, et deux ailettes telles que 5 de prise digitale.

Le dispositif d'injection comprend, en outre, une bague 6 adaptée pour venir coiffer le tronçon d'extrémité du tube cylindrique 1 opposé à l'embout 3.

Cette bague 6 se compose longitudinalement de deux manchons cylindriques 6a, 6b de diamètres externe et interne différents, séparés par des épaulements respectivement externe 7 et interne 8.

Le premier manchon 6a, de plus grands diamètres, est adapté pour venir s'emmancher sur sa plus grande longueur sur le tronçon d'extrémité du tube cylindrique 1 du corps de seringue. Intérieurement, ce manchon 6a présente un alésage 9 divisé longitudinalement en quatre tronçons consistant successivement en :
- un premier tronçon d'extrémité 9a destiné à être présenté en regard de l'extrémité du corps de seringue lors du montage de la bague 6, et présentant une forme tronconique définissant une rampe apte à faciliter ce montage,
- un premier tronçon intermédiaire 9b de forme cylindrique de diamètre interne conjugué du diamètre externe du tube cylindrique 1,
- un deuxième tronçon intermédiaire 9c de forme torique adapté pour venir s'encliqueter sur la collerette 2 du tube cylindrique 1,
- et un quatrième tronçon 9d, de jonction avec le deuxième manchon 6b, de forme cylindrique de diamètre interne légèrement inférieur à celui du premier tronçon intermédiaire 9b.

Le second manchon 6b, de plus faibles diamètres, présente quant à lui, intérieurement, un alésage cylindrique 10 séparé, tel que précité, du tronçon de jonction 9d du premier manchon 6a par l'épaulement interne 8.

Ce second manchon 6b est obturé, à l'opposé du premier manchon 6a, par une paroi frontale 11 percée d'un orifice circulaire axial 12 de diamètre inférieur à celui de l'alésage 10.

Cette paroi frontale 11 comporte deux échancrures 13, 14 diamétralement opposées ménagées sur le pourtour de l'orifice axial 12, et définissant avec ce dernier une fente rectiligne d'indexage de l'orientation du capuchon de protection lors du montage du dispositif d'injection.

Par ailleurs, le second manchon 6b présente également deux rainures internes longitudinales 15, 16, diamétralement opposées, s'étendant chacune à l'aplomb d'une échancrure 13, 14.

Chacune de ces rainures 15, 16 de largeur constante sur sa plus grande longueur présente, en outre, un tronçon d'extrémité 20 de jonction avec l'alésage 9 du premier manchon 6a, de largeur croissante, de façon à former un entonnoir d'entrée.

Ce second manchon 6b comporte, enfin, en saillie par rapport au fond de chacune de ces rainures 15, 16, un cran tel que 17 présentant une section longitudinale triangulaire.

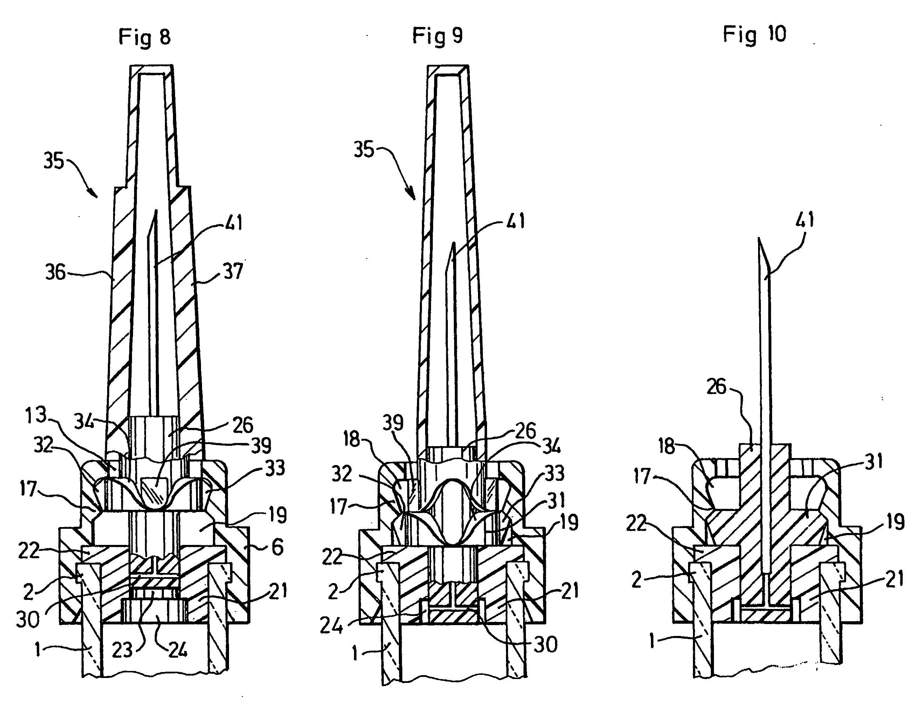

Tel que représenté aux figures 8 à 10, si l'on considère la bague 6 vue en coupe par un plan longitudinal axial coupant ladite bague au niveau des rainures 15, 16, le second manchon 6b est ainsi divisé intérieurement en deux chambres 18, 19 juxtaposées longitudinalement et séparées l'une de l'autre par les crans 17.

Le dispositif d'injection comporte, en outre, des moyens d'obturation de l'extrémité du tube cylindrique 1 du corps de seringue opposée à l'embout 3, aptes à permettre d'autoriser l'injection du liquide renfermé dans ledit tube.

Ces moyens d'obturation comprennent, en premier lieu, un bouchon cylindrique 21 apte à être inséré dans le tube cylindrique 1 et doté d'une collerette cylindrique 22 de butée sur l'extrémité dudit tube.

Ce bouchon 21 est, en outre, percé d'un alésage cylindrique traversant 23 débouchant dans un lamage 24 ménagé dans la face frontale dudit bouchon opposée à la collerette 22.

Ces moyens d'obturation comprennent, en outre, un organe d'obturation et de distribution 25 se présentant sous la forme d'un fût cylindrique de diamètre externe adapté pour venir se loger dans l'alésage 23 du bouchon 21.

Ce fût 26 est, en outre, percé sur sa plus grande longueur d'un alésage axial longitudinal 27 débouchant dans une fraisure 28 ménagée dans une des faces frontales dudit fût, ledit alésage étant destiné de façon classique à recevoir une aiguille d'injection 41.

Ce fût 26 est également percé, dans le prolongement de l'alésage 27, d'un conduit axial 29 de liaison dudit alésage avec un conduit transversal traversant 30 ménagé à faible distance de la face frontale dudit fût opposée à celle dans laquelle est ménagée la fraisure 28.

Cet organe d'obturation et de distribution 25 comprend par ailleurs un collet intermédiaire 31 consistant en un disque de forme générale cylindrique de diamètre conjugué du diamètre interne de l'alésage 10 du second manchon 6b de la bague 6.

Ce collet intermédiaire 31 destiné à pouvoir se loger dans l'une ou l'autre chambre 18, 19 de la bague 6, et à pouvoir se déplacer axialement dans les deux sens de l'une vers l'autre desdites chambres, présente à cet effet deux ergots diamétralement opposés 32, 33, de section longitudinale sensiblement triangulaire conjuguée de celle des tronçons des rainures 15, 16 matérialisant ces chambres 18, 19, et délimités par les crans 17.

En dernier lieu, ce collet intermédiaire 31 comporte une face frontale supérieure dans laquelle sont ménagés deux évidements tels que 34 de forme concave présentant un axe de symétrie longitudinal, chacun desdits évidements étant ménagé de façon que ledit axe de symétrie soit décalé d'un angle α égal à 90° par rapport aux ergots 32, 33.

Le dispositif d'injection comporte; enfin, un capuchon de protection 35 adapté pour venir coiffer l'aiguille d'injection 41.

Ce capuchon de protection 35 présente une forme générale tronconique de diamètre de base adapté pour pouvoir s'étendre au travers de l'orifice axial 12 ménagé dans la paroi frontale 11 de la bague 6.

Ce capuchon de protection 35 comporte, en outre, deux ailettes longitudinales 36, 37 diamétralement opposées définissant un diamètre maximal hors tout dudit capuchon, dans l'axe desdites ailettes, légèrement supérieur au diamètre séparant le fond des échancrures 13, 14 ménagées dans la paroi frontale de la bague 6.

Ces ailettes 36, 37 sont par ailleurs interrompues à distance de l'extrémité inférieure ouverte du capuchon de protection 35, de façon, tel que représenté à la figure 8, à venir buter axialement sur la paroi frontale 11 de la bague 6 une fois le dispositif d'injection assemblé.

Ce capuchon de protection 35 comporte, en outre, dans le prolongement de son extrémité ouverte, deux oreilles telles que 38 diamétralement opposées, en forme d'ondulation convexe complémentaire des profils concaves des évidements 34 du collet intermédiaire 31 de l'organe d'obturation et de distribution 25.

De plus, ce capuchon de protection 35 comporte, au niveau de chaque oreille 38, un pion radial externe 39, 40.

Les opérations d'assemblage de ce dispositif d'injection sont décrites ci-dessous.

En premier lieu, le capuchon de protection 35 est assemblé avec l'organe d'obturation et de distribution 25, de façon à amener à coopérer et à emboîter les profils concaves et convexes respectifs des évidements 34 et des oreilles 38.

Ensuite, l'ensemble précité est introduit à l'intérieur de la bague 6, le positionnement relatif en rotation de ladite bague et dudit ensemble étant indexé grâce aux ailettes 36, 37 amenées à coulisser dans les échancrures 13, 14.

Cette introduction conduit d'une part à amener les ailettes 36, 37 de façon qu'elles s'étendent entièrement à l'extérieur de la bague 6, dans le prolongement de la paroi frontale 11 de cette dernière, et d'autre part, à positionner le collet intermédiaire 31 dans la chambre supérieure 18 de la bague 6.

Le bouchon 21 est ensuite également mis en place dans la bague 6, dans une position où la collerette 22 dudit bouchon vient se loger dans le quatrième tronçon 9d du premier manchon 6a de la bague 6.

Dans cette position du bouchon 21, et tel que représenté à la figure 8, le conduit transversal 30 du fût 26 de l'organe d'obturation et de distribution 25 se trouve positionné dans l'alésage 23 dudit bouchon, en retrait par rapport au lamage 24 de ce dernier. De plus, ce bouchon 21 obture frontalement le compartiment inférieur 19 de la bague 6 de façon à limiter vers le bas le déplacement axial de l'organe d'obturation et de distribution 25.

La dernière opération consiste enfin à présenter le corps de seringue en regard des éléments pré-assemblés ci-dessus décrits, et à emmancher ledit corps de seringue à l'intérieur de la bague 6 jusqu'à amener la collerette 2 du tube cylindrique 1 à se clipser dans le tronçon intermédiaire torique 9c du premier manchon 6a de ladite bague.

Une fois cet assemblage réalisé, le capuchon de protection 35 ne peut pas être repoussé à l'intérieur de la bague du fait de la disposition des ailettes 36, 37 positionnées en butée contre la paroi frontale 11 de ladite bague. De plus, ce capuchon de protection 35 ne peut pas être retiré sans avoir subi au préalable un mouvement de rotation, du fait de la présence des pions radiaux 39, 40.

De ce fait, après remplissage du corps de seringue et obturation de ce dernier par un corps de piston de type classique, le dispositif d'injection peut alors être manipulé, stocké, ..., sans risque d'écoulement de liquide ni de contamination de ce liquide.

Enfin, en vue d'une injection, et tel que représenté à la figure 9, il suffit à l'utilisateur de faire tourner le capuchon de protection 35 d'un quart de tour dans l'un ou l'autre sens, et de tirer sur ce dernier de façon à le dissocier du corps de seringue.

Lors de ce retrait, et en premier lieu, l'organe d'obturation et de distribution 25 est amené automatiquement à se déplacer axialement vers une position d'injection où, d'une part le collet intermédiaire 31 vient se loger dans la chambre inférieure 19 de la bague 6, et d'autre part, le conduit transversal 30 débouche dans le lamage 24 du bouchon 21 autorisant l'écoulement et l'injection du liquide.

De plus, les pions radiaux 39, 40 du capuchon de protection 35 sont amenés automatiquement à l'aplomb des échancrures 13, 14 de la bague 6, autorisant le retrait effectif dudit capuchon de protection.

## Revendications

1. Dispositif d'injection comprenant un corps de seringue (1, 3, 21) délimitant une chambre destinée à être remplie d'un liquide, notamment médicamenteux, et un organe (25) d'obturation de la chambre et de distribution du liquide, comportant un embout d'obturation de ladite chambre, d'une part prolongé d'une embase dans laquelle est scellée une aiguille (41) coiffée par un capuchon de protection (35), et d'autre part, percé dans le prolongement de ladite aiguille d'un conduit (29, 30) de distribution du liquide, ledit organe d'obturation et de distribution (25) étant mobile axialement entre une position, dite position d'obturation, où le conduit de distribution (29, 30) est isolé de la chambre du corps de seringue (1, 3, 21), et une position, dite position d'injection, où ledit conduit de distribution communique avec ladite chambre:
- l'organe d'obturation et de distribution (25) comprenant un collet intermédiaire (31) disposé entre l'embout d'obturation et l'embase,
- ledit dispositif d'injection comprenant une bague (6) dotée d'une paroi frontale (11) percée d'un orifice axial (12) pour le passage du capuchon de protection (35), ladite bague étant adaptée pour coiffer un des tronçons d'extrémité du corps de seringue (1, 3, 21) et pour délimiter, dans le prolongement dudit tronçon d'extrémité, deux compartiments internes (18, 19) juxtaposés axialement, chacun de forme conjuguée du collet intermédiaire (31) de l'organe d'obturation et de distribution (25), apte à loger ledit collet intermédiaire, lesdits compartiments étant séparés par des organes de butée axiale (17) aptes à autoriser un déplacement axial du collet intermédiaire (31) d'un compartiment (18, 19) vers l'autre compartiment (19, 18),
- la bague (6) et le collet intermédiaire (31) comprenant des organes conjugués (15, 16, 32, 33), respectivement interne et externe, de blocage relatif en rotation desdites bague et collet intermédiaire,
- le capuchon de protection (35) comprenant un tronçon d'extrémité adapté pour s'étendre au travers de l'orifice axial (12) de la paroi frontale (11) de la bague (6), de façon à coiffer l'embase et à venir axialement en butée contre le collet intermédiaire (31) de l'organe d'obturation et de distribution (25),
- le tronçon d'extrémité du capuchon de protection (35) et le collet intermédiaire (31) de l'organe d'obturation et de distribution (25) présentant frontalement, en regard, des profils conjugués (34, 38) en forme de came, aptes à engendrer un déplacement axial de l'organe d'obturation et de distribution (25) de sa position d'obturation vers sa position d'injection, lors d'un mouvement de rotation du capuchon de protection (35),
- le capuchon de protection (35) comportant des organes externes (36, 37) agencés pour venir en butée axiale sur la paroi frontale (11) de la bague (6), dans une position où ledit capuchon coiffe l'embase de l'organe d'obturation et de distribution (25).

2. Dispositif d'injection selon la revendication 1, **caractérisé en ce que** les profils en forme de came (34, 38) du collet intermédiaire (31) de l'organe d'obturation et de distribution (25) et du tronçon d'extrémité du capuchon de protection (35) présentent respectivement une forme concave et une forme convexe conjuguées.

3. Dispositif d'injection selon l'une des revendications 1 ou 2, **caractérisé en ce que** :
- le tronçon d'extrémité du capuchon de protection (35) comporte un pion externe radial (39, 40) au niveau d'au moins un des profils (38) en forme de came,
- la bague (6) comporte, pour chaque pion externe (39, 40) du capuchon de protection (35), une échancrure (13, 14) ménagée sur le pourtour de l'orifice axial (12) de la paroi frontale (11), ladite échancrure étant ménagée de façon à être décalée angulairement d'un angle (a) par rapport au pion externe (39, 40), dans la position d'obturation de l'organe de distribution et d'obturation (25), et pour se trouver à l'aplomb dudit pion externe dans la position d'injection dudit organe d'obturation et de distribution.

4. Dispositif d'injection selon la revendication 3, **caractérisé en ce que** chaque profil (34, 38) en forme de came présente deux pentes s'étendant symétriquement de part et d'autre d'un axe de symétrie longitudinal, de façon à autoriser une rotation du capuchon de protection (35) dans l'un ou l'autre sens de rotation.

5. Dispositif d'injection selon l'une des revendications 3 ou 4, **caractérisé en ce que** l'angle (α) est sensiblement égal à 90°.

6. Dispositif d'injection selon l'une des revendications 3 à 5, **caractérisé en ce que** le capuchon de protection (35) comporte, pour chaque échancrure (13, 14) de la paroi frontale (11) de la bague (6), une ailette longitudinale (36, 37) décalée angulairement d'un angle (α) par rapport à chaque pion externe (39, 40) dudit capuchon de protection, apte à pouvoir coulisser dans ladite échancrure lorsque ce capuchon de protection (35) est présenté en regard de l'intérieur de la bague (6).

7. Dispositif d'injection selon la revendication 6, **caractérisé en ce que** chaque ailette longitudinale (36, 37) du capuchon de protection (35) est interrompue à distance de l'extrémité du tronçon d'extrémité dudit capuchon de protection, et présente une épaisseur adaptée pour venir en butée axiale sur la paroi frontale (11) de la bague (6).

8. Dispositif d'injection selon la revendication 7, **caractérisé en ce que** chaque ailette (36, 37) du capuchon de protection (35) comprend une face externe latérale inclinée longitudinalement de façon à former une rampe apte à permettre à ladite ailette de franchir l'échancrure (13, 14) de la paroi frontale (11) de la bague (6) en fin d'introduction dudit capuchon de protection à l'intérieur de ladite bague.

9. Dispositif d'injection selon l'une des revendications précédentes, **caractérisé en ce que** :
- la bague (6) délimite un logement interne de forme cylindrique et comporte au moins une rainure interne longitudinale (15, 16) par rapport au fond de laquelle fait saillie un cran (17) ménagé en position intermédiaire le long de ladite rainure, ledit cran présentant longitudinalement une face externe en forme de dièdre apte à faire office d'organe de butée axiale séparant les deux compartiments internes (18, 19) de ladite bague,
- le collet intermédiaire (31) de l'organe d'obturation et de distribution (25) comprend, pour chaque rainure (15, 16) de la bague (6), un ergot latéral (32, 33) apte à venir se loger dans ladite rainure, et de forme conjuguée des compartiments (18, 19) délimités par le cran (17) s'étendant dans cette dernière.

10. Dispositif d'injection selon l'une des revendications précédentes, **caractérisé en ce qu'**il comprend :
- un bouchon d'obturation (21) de dimensions adaptées pour être inséré dans le corps de seringue (1, 3), percé d'un alésage traversant (23) débouchant dans ledit corps de seringue au niveau d'un lamage (24).
- l'organe d'obturation et de distribution (25) présente des dimensions adaptées pour être inséré dans l'alésage (23) du bouchon d'obturation (21), et comprend un conduit de distribution (29, 30) comportant une branche transversale (30) ménagée de façon à être positionnée en retrait du lamage (24) dudit bouchon d'obturation, dans la position d'obturation de l'organe d'obturation et de distribution (25), et pour s'étendre dans ledit lamage dans la position d'injection dudit organe.

## Patentansprüche

1. Injektionsvorrichtung, umfassend einen Spritzenkörper (1, 3, 21), der eine mit einer Flüssigkeit, insbesondere einem Medikament, zu füllende Kammer begrenzt, und einen Mechanismus (25) zum Verschließen der Kammer und zum Ausgeben der Flüssigkeit, der ein Ansatzstück zum Verschließen der genannten Kammer umfasst, auf der einen Seite verlängert durch einen Sitz, in den eine Nadel (41) eingegossen ist, die von einer Schutzkappe (35) umgeben ist, und auf der anderen Seite in der Verlängerung der genannten Nadel von einem Kanal (29, 30) zum Ausgeben der Flüssigkeit durchbohrt, wobei der genannte Verschluss- und Ausgabemechanismus (25) axial zwischen einer Position, Verschlussposition genannt, in der der Ausgabekanal (29, 30) von der Kammer des Spritzenkörpers (1, 3, 21) isoliert ist, und einer Position, Injektionsposition genannt, beweglich ist, in der der genannte Ausgabekanal mit der genannten Kammer in Verbindung ist;
- wobei der Verschluss- und Ausgabemechanismus (25) eine Zwischenhülse (31) aufweist, die zwischen dem Verschlussansatzstück und dem Sitz angeordnet ist,
- wobei die genannte Injektionsvorrichtung einen Ring (6) aufweist, der mit einer Frontwand (11) versehen ist, die von einer axialen Öffnung (12) für die Passage der Schutzkappe (35) durchbohrt ist, wobei der genannte Ring so gestaltet ist, dass er einen der Endabschnitte des Spritzenkörpers (1, 3, 21) umgibt, und so, dass er in der Verlängerung des genannten Endabschnitts zwei axial nebeneinander liegende Innenkammern (18, 19) begrenzt, jede mit einer Form, die zur Zwischenhülse (31) des Verschlussund Ausgabemechanismus (25) passt und die genannte Zwischenhülse aufnehmen kann, wobei die genannten Kammern durch axiale Anschlagelemente (17) getrennt sind, die so gestaltet sind, dass sie eine axiale Verschiebung der Zwischenhülse (31) der einen Kammer (18, 19) in Richtung auf die andere Kammer (19, 18) zulassen,
- wobei der Ring (6) und die Zwischenhülse (31) zusammenpassende Elemente (15, 16, 32, 33), jeweils innen und außen, zum relativen rotationalen Sperren des genannten Rings und der genannten Zwischenhülse umfassen,
- wobei die Schutzkappe (35) einen Endabschnitt umfasst, der so gestaltet ist, dass er durch die axiale Öffnung (12) der Frontwand (11) des Rings (6) passt, um den Sitz zu umgeben und um axial in Anlage an der Zwischenhülse (31) des Verschluss- und Ausgabemechanismus (25) zu kommen,
- wobei der Endabschnitt der Schutzkappe (35) und die Zwischenhülse (31) des Verschluss- und Ausgabemechanismus (25) vorne einander gegenüber zusammenpassende Profile (34, 38) in Nockenform aufweisen, die eine axiale Verschiebung des Verschluss- und Ausgabemechanismus (25) von seiner Verschlussposition in Richtung auf seine Injektionsposition während einer Rotationsbewegung der Schutzkappe (35) bewirken können,
- wobei die Schutzkappe (35) externe Elemente (36, 37) umfasst, die so gestaltet sind, dass sie in axiale Anlage an der Frontwand (11) des Rings (6) in einer Position kommen, in der die genannte Kappe den Sitz des Verschlussund Ausgabemechanismus (25) umgibt.

2. Injektionsvorrichtung nach Anspruch 1, **dadurch gekennzeichnet, dass** die Profile in Nockenform (34, 38) der Zwischenhülse (31) des Verschluss- und Ausgabemechanismus (25) und des Endabschnitts der Schutzkappe (35) jeweils eine konkave Form und eine dazu passende konvexe Form haben.

3. Injektionsvorrichtung nach Anspruch 1 oder 2, **dadurch gekennzeichnet, dass**:
- der Endabschnitt der Schutzkappe (35) ein radiales Außenstück (39, 40) in der Höhe von wenigstens einem der Profile (38) in Nockenform umfasst,
- der Ring (6) für jedes Außenstück (39, 40) der Schutzkappe (35) einen bogenförmigen Ausschnitt (13, 14) aufweist, der auf dem Umfang der axialen Öffnung (12) der Frontwand (11) vorgesehen ist, wobei der genannte bogenförmige Ausschnitt (13, 14) so gestaltet ist, dass er winkelmäßig in einem Winkel (α) in Bezug auf das Außenstück (39, 40) in der Verschlussposition des Verschluss- und Ausgabemechanismus (25) versetzt ist, und so, dass er sich lotrecht zu dem genannten Außenstück in der Injektionsposition des genannten Verschluss- und Ausgabemechanismus befindet.

4. Injektionsvorrichtung nach Anspruch 3, **dadurch gekennzeichnet, dass** jedes Profil (34, 38) in Nockenform zwei Neigungen aufweisen, die symmetrisch auf beiden Seiten einer Längssymmetrieachse verlaufen, um eine Rotation der Schutzkappe (35) in der einen oder der anderen Rotationsachse zu ermöglichen.

5. Injektionsvorrichtung nach Anspruch 3 oder 4, **dadurch gekennzeichnet, dass** der Winkel (α) im Wesentlichen 90° beträgt.

6. Injektionsvorrichtung nach einem der Ansprüche 3 bis 5, **dadurch gekennzeichnet, dass** die Schutzkappe (35) für jeden bogenförmigen Ausschnitt (13, 14) der Frontwand (11) des Rings (6) einen Längsflügel (36, 37) aufweist, der winkelmäßig um einen Winkel (α) in Bezug auf jedes Außenstück (39, 40) der genannten Schutzkappe versetzt ist, so dass er in dem genannten bogenförmigen Ausschnitt gleiten kann, wenn die Schutzkappe (35) an der Innenseite des Rings (6) angehalten wird.

7. Injektionsvorrichtung nach Anspruch 6, **dadurch gekennzeichnet, dass** jeder Längsflügel (36, 37) der Schutzkappe (35) in einem Abstand vom Ende des Endabschnitts der genannten Schutzkappe unterbrochen ist und eine solche Dicke aufweist, dass sie an der Frontwand (11) des Rings (6) zur axialen Anlage kommt.

8. Injektionsvorrichtung nach Anspruch 7, **dadurch gekennzeichnet, dass** jeder Flügel (36, 37) der Schutzkappe (35) eine laterale Außenfläche aufweist, die longitudinal so geneigt ist, dass eine Rampe entsteht, die es dem genannten Flügel gestattet, den bogenförmigen Ausschnitt (13, 14) der Frontwand (11) des Rings (6) am Ende der Einführung der genannten Schutzkappe ins Innere des genannten Rings zu passieren.

9. Injektionsvorrichtung nach einem der vorherigen Ansprüche, **dadurch gekennzeichnet, dass**:
- der Ring (6) eine interne Aufnahme mit einer zylindrischen Form begrenzt und wenigstens eine interne Längsrille (15, 16) aufweist, in Bezug auf deren Boden ein Vorsprung (17) vorsteht, der in einer Zwischenposition auf der genannten Rille vorgesehen ist, wobei der genannte Vorsprung longitudinal eine Außenfläche in der Form eines Dieders hat, die als axiales Anlageelement dient, das die beiden Innenkammern (18, 19) des genannten Rings trennt,
- die Zwischenhülse (31) des Verschluss- und Ausgabemechanismus (25) für jede Rille (15, 16) des Rings (6) einen seitlichen Ansatz (32, 33) aufweist, der in der genannten Rille aufgenommen wird und eine Form hat, die zu den Kammern (18, 19) passt, die durch.den in dieser verlaufenden Vorsprung (17) begrenzt wird.

10. Injektionsvorrichtung nach einem der vorherigen Ansprüche, **dadurch gekennzeichnet, dass** sie Folgendes umfasst:
- eine Verschlusskappe (21) mit solchen Abmessungen, dass sie in den Spritzenkörper (1, 3) eingeführt werden kann, der von einer Durchgangsbohrung (23) durchbohrt ist, die in dem genannten Spritzenkörper in der Höhe einer Senkung (24) mündet;
- den Verschluss- und Ausgabemechanismus (25) mit solchen Abmessungen, dass er in die Bohrung (23) der Verschlusskappe (21) eingeführt werden kann, und der einen Ausgabekanal (29, 30) umfasst, der einen Querzweig (30) aufweist, der so gestaltet ist, dass er in der Verschlussposition des Verschluss- und Ausgabemechanismus (25) so in die Senkung (24) der genannten Verschlusskappe eingelassen werden kann, dass er in der genannten Senkung in der Injektionsposition des genannten Mechanismus verläuft.

## Claims

1. Injection device comprising a syringe body (1, 3, 21), which delimits a chamber which is designed to be filled with a liquid, in particular a medicinal liquid, and a unit (25) for closing off the chamber and distributing the liquid, comprising an end piece for closing off the said chamber, which is firstly extended by a base, in which there is sealed a needle (41) which is covered by a protective cap (35), and secondly, which is perforated in the extension of the said needle by a duct (29, 30) for distribution of the liquid, the said unit (25) for closing off and distributing being mobile axially between a position known as the closing-off position, in which the distribution duct (29, 30) is isolated from the chamber of the syringe body (1, 3, 21), and a position known as the injection position, in which the said distribution duct communicates with the said chamber,
- wherein the unit (25) for closing off and distributing comprises an intermediate collar (31), which is disposed between the closing-off end piece and the base;
- wherein the said injection device comprises a ring (6) which is provided with a front wall (11), in which there is provided an axial opening (12) for passage of the protective cap (35), the said ring being adapted to cover one of the end sections of the syringe body (1, 3, 21) and to delimit, in the extension of the said end section, two inner compartments (18, 19) which are juxtaposed axially, each of which has a shape which is conjugated with the intermediate collar (31) of the unit (25) for closing off and distributing, and can accommodate the said intermediate collar, the said compartments being separated by axial stop units (17) which can permit axial displacement of the intermediate collar (31) of one compartment (18,19) towards the other compartment (19, 18);
- wherein the ring (6) and the intermediate (31) collar comprise respectively inner and outer conjugated units (15, 16, 32,33), for relative locking in rotation of the said ring and intermediate collar;
- wherein the protective cap (35) comprises an end section, which is designed to extend through the axial opening (12) in the front wall (11) of the ring (6), such as to cover the base and abut axially the intermediate collar (31) of the unit for closing off and distributing (25);
- wherein the end section of the protective cap (35), and the intermediate collar (31) of the unit (25) for closing off and distributing have at the front, and opposite, conjugated profiles (34, 38) in the form of a cam, which can give rise to axial displacement of the unit (25) for closing off and distributing, from its closing-off position towards its injection position, during movement of rotation of the protective cap (35);
- wherein the protective cap (35) comprises outer units (36, 37) which are designed to abut axially the front wall (11) of the ring (6), in a position in which the said cap covers the base of the unit (25) for closing off and distributing.

2. Injection device according to claim 1, **characterised in that** the profiles in the form of a cam (34, 38), of the intermediate collar (31) of the unit (25) for closing off and distributing, and of the end section of the protective cap (35), have respectively a conjugated concave shape and a convex shape.

3. Injection device according to claim 1 or claim 2, **characterised in that**:
- the end section of the protective cap (35) comprises a radial outer pin (39, 40) at the level of at least one of the profiles (38) in the form of a cam;
- for each outer pin (39, 40) of the protective cap (35), the ring (6) comprises a cutout (13, 14) which is provided in the periphery of the axial opening (12) of the front wall (11), the said cutout being such as to be offset angularly by an angle (α) relative to the outer pin (39, 40), in the closing-off position of the unit (25) for closing off and distributing, and to be plumb with the said outer pin in the injection position of the said unit for closing off and distributing.

4. Injection device according to claim 3, **characterised in that** each profile (34, 38) in the form of a cam has two slopes, which extend symmetrically on both sides of a longitudinal axis of symmetry, such as to permit rotation of the protective cap (35) in either direction of rotation.

5. Injection device according to claim 3 or claim 4, **characterised in that** the angle (α) is substantially equivalent to 90°.

6. Injection device according to any one of claims 3 to 5, **characterised in that** for each cutout (13, 14) in the front wall (11) of the ring (6), the protective cap (35) comprises a longitudinal fin (36, 37), which is offset angularly by an angle (α) relative to each outer pin (39, 40) of the said protective cap, which can slide in the said cutout when this protective cap (35) is presented opposite the interior of the ring (6).

7. Injection device according to claim 6, **characterised in that** each longitudinal fin (36, 37) of the protective cap (35) is interrupted at a distance from the end of the end section of the said protective cap, and has a thickness which is designed to abut axially the front wall (11) of the ring (6).

8. Injection device according to claim 7, **characterised in that** each fin (36, 37) of the protective cap (35) comprises a lateral outer surface which is inclined longitudinally, such as to form a ramp, which can allow the said fin to clear the cutout (13, 14) in the front wall (11) of the ring (6), on completion of introduction of the said protective cap inside the said ring.

9. Injection device according to any one of the preceding claims, **characterised in that**:
- the ring (6) delimits an inner receptacle which has a cylindrical shape, and comprises at least one longitudinal inner groove (15, 16), relative to the base of which there projects a catch (17), provided in an intermediate position along the said groove, which catch has longitudinally an outer surface in the form of a dihedron, which can act as an axial stop unit separating the two inner compartments (18, 19) of the said ring;
- for each groove (15, 16) in the ring (6), the intermediate collar (31) of the unit (25) for closing off and distributing comprises a lateral lug (32, 33), which can be accommodated in the said groove, and has a shape which is conjugated with the compartments (18, 19) delimited by the catch (17) which extends in the latter.

10. Injection device according to any one of the preceding claims, **characterised in that** it comprises:
- a closing-off stopper (21) with dimensions which are suitable for being inserted in the syringe body (1, 3), provided with a through bore (23) which opens into the said syringe body at a countersink (24);
- the unit (25) for closing off and distributing has dimensions which are designed to be inserted in the bore (23) in the closing-off stopper (21), and comprises a distribution duct (29, 30) containing a transverse branch (30), which is disposed such as to be positioned recessed from the countersink (24) in the said closing-off stopper, in the closing-off position of the unit (25) for closing off and distributing, and to extend into the said countersink, in the injection position of the said unit.
